# EUROPEAN PATENT APPLICATION

(11) **EP 3 587 559 A1**
(43) Date of publication of application: **01.01.2020**
(21) Application number: 18757824.0
(22) Date of filing: 22.02.2018
(51) Int. Cl.: C12N 5/07

(54) **PRODUCTION METHOD FOR ARTIFICIAL PLURIPOTENT STEM CELLS**

(30) Priority: 24.02.2017 US 201762463420 P
(71) Applicant: Tanabe, Koji, Palo Alto, CA 94303 (US); I Peace, Inc., Palo Alto, CA 94303 (US)
(72) Inventor: TANABE, Koji, Palo Alto California 94303 (US); SUTO, Kenta, Palo Alto California 94303 (US); SHIMODA, Hidenori, Palo Alto California 94303 (US); KELLY, Brendan, Palo Alto California 94303 (US)
(74) Representative: MacLean, Martin Robert
(86) International application number: PCT/JP2018/006588
(87) International publication number: WO 2018/155595

(57) **Abstract**

The present disclosure provides a production method for artificial pluripotent stem cells, the method comprising preparing somatic cells, and introducing a Sendai virus including reprogramming factor RNA into the somatic cell. The present disclosure also provides a production method for artificial pluripotent stem cells, the method comprising preparing somatic cells, introducing reprogramming factor RNA into the somatic cells using an RNA transfection reagent, and reprogramming the somatic cells in a gel culture medium.

## Description

### FIELD

The present invention relates to cell technology and to a production method for artificial pluripotent stem cells.

### BACKGROUND

Artificial pluripotent stem (iPS) cells are cells with two distinctive abilities. One is the ability to change into various cells that make up the body. The other is the ability to proliferate semipermanently. Because iPS cells have these two abilities, iPS cells produced from autologous somatic cells can be changed to the desired somatic cells and used in transplant therapy without rejection. Thus, iPS cells are considered to be a powerful technology in regenerative medicine.

From the birth of iPS cells to the present day, numerous production methods have been established. Exemplary iPS cell production methods include methods using retroviruses/lentiviruses and methods using episomal vectors.

Methods using retroviruses/lentiviruses will be explained. A gene encoding a reprogramming factor can be introduced into a cell by infecting a somatic cell with a retrovirus or lentivirus. Furthermore, retroviruses and lentiviruses can insert reprogramming factors into the genome of somatic cells and induce stable expression of the reprogramming factors in cells.

However, methods using retroviruses/lentiviruses have the following problems. First, the insertion of reprogramming factors into the genome of somatic cells damages existing genes or promoters and might cause cell carcinogenesis. Further, reprogramming factors inserted into the genome might be reactivated after iPS cells have been converted to somatic cells. Thus, there is a risk of carcinogenesis in cells for transplantation derived from iPS cells. In mouse models, reactivation of introduced reprogramming factors has actually been observed in somatic cells and carcinogenesis has been confirmed (e.g. refer to NPL 1).

Episomal vectors are circular DNA which self-amplify in the nucleus. Episomal vectors are, in principle, considered not to be incorporated into the genome. However, there have been reports of recent research, which state that inserted episomal vector fragments are interspersed in the genome of iPS cells created with episomal vectors. Thus, there is the problem that reprogramming genes may remain in cells. For example, if c-MYC or KLF4 remains in cells, this could lead to carcinogenesis. Moreover, in order to test whether any reprogramming genes are remaining in a cell requires substantial costs, and it is impossible to demonstrate that none of the cells of the transplant cell pool have episomal plasmids inserted into the genome or remaining in the cells.

As the methods using retroviruses/lentiviruses and methods using episomal vectors have the aforementioned problems, a production method for iPS cells using RNA is proposed (e.g. NPL 2).

### [CITATION LIST]

### [NON-PATENT LITERATURE]

[NPL 1] Nature 448, 313-317
[NPL 2] Nature Biotechnol 26(3): 313-315, 2008.

### SUMMARY

### [TECHNICAL PROBLEM]

A method for introducing reprogramming factor RNA efficiently into somatic cells is desired. One object of the present invention is to provide an efficient production method for an artificial pluripotent stem cell that is safe and suitable for clinical use.

### [SOLUTION TO PROBLEM]

According to an aspect of the present invention there is provided a production method for an artificial pluripotent stem cell, comprising preparing a somatic cell and introducing a Sendai virus comprising reprogramming factor RNA into the somatic cell.

In the production method for an artificial pluripotent stem cell, a Sendai virus comprising reprogramming factor RNA may be introduced into a somatic cell which is suspension-cultured in a gel medium.

In the production method for an artificial pluripotent stem cell, a Sendai virus comprising reprogramming factor RNA may be introduced into a somatic cell which is adhesion-cultured.

The production method for an artificial pluripotent stem cell may further comprise reprogramming a somatic cell in a gel medium.

In the production method for an artificial pluripotent stem cell, the somatic cell may be a fibroblast.

In the production method for an artificial pluripotent stem cell, the somatic cell may be a blood cell.

The production method for an artificial pluripotent stem cell may further comprise separating a monocyte as the somatic cell using an erythrocyte separating agent.

The production method for an artificial pluripotent stem cell may further comprise separating a monocyte as the somatic cell using a filter.

The production method for an artificial pluripotent stem cell may further comprise separating a monocyte as the somatic cell using an immunomagnetic bead.

In the production method for an artificial pluripotent stem cell, reprogramming factor RNA may comprise M₃O or OCT3/4 mRNA, SOX2 mRNA, KLF4 mRNA, and c-MYC mRNA.

In the production method for an artificial pluripotent stem cell, the gel culture medium need not be stirred. The gel culture medium need not contain a growth factor. The gel culture medium may contain a growth factor at a concentration of 40% by mass or less. The gel culture medium need not contain bFGF.

According to another aspect of the present invention, there is provided a production method for an artificial pluripotent stem cell, comprising preparing a somatic cell and introducing reprogramming factor RNA into the somatic cell using an RNA transfection reagent and reprogramming the somatic cell in gel culture medium.

In the production method for an artificial pluripotent stem cell, the somatic cell may be a fibroblast.

In the production method for an artificial pluripotent stem cell, the somatic cell may be a blood cell.

The production method for an artificial pluripotent stem cell may further comprise separating a monocyte as a somatic cell using an erythrocyte separating agent.

The production method for an artificial pluripotent stem cell may further comprise separating a monocyte as a somatic cell using a filter.

The production method for an artificial pluripotent stem cell may further comprise separating a monocyte as a somatic cell using an immunomagnetic bead.

In the production method for an artificial pluripotent stem cell, reprogramming factor RNA may comprise M₃O or OCT3/4 mRNA, SOX2 mRNA, KLF4 mRNA, and c-MYC mRNA.

In the production method for an artificial pluripotent stem cell, the gel culture medium need not be stirred. The gel culture medium need not contain a growth factor. The gel culture medium may contain a growth factor at a concentration of 40% by mass or less. The gel culture medium need not contain bFGF.

In the production method for an artificial pluripotent stem cell, the introduction of reprogramming factor RNA may be carried out a plurality of times.

### [ADVANTAGEOUS EFFECTS OF INVENTION]

According to the present invention it is possible to provide an efficient production method for safe artificial pluripotent stem cells suitable for clinical use.

### BRIEF DESCRIPTION OF DRAWINGS

FIG. 1 shows fluorescent microscope photographs and flow cytometry dot-plots of the results of Example 1 of the first embodiment.
FIG. 2 shows fluorescent microscope photographs and flow cytometry dot-plots of the results of Example 1 of the first embodiment.
FIG. 3 shows photographs of iPS cell colonies in Example 3 of the first embodiment.
FIG. 4 shows photographs of immunostained cells in Example 3 of the first embodiment.
FIG. 5 shows photographs of immunostained cells in Example 4 of the first embodiment.
FIG. 6 shows photographs of immunostained cells in Example 5 of the first embodiment.
FIG. 7 shows photographs of immunostained cells in Example 6 of the first embodiment.
FIG. 8 shows flow cytometry dot-plots of the results of Examples 7 to 9 of the first embodiment.
FIG. 9 shows photographs of an iPS cell colony in Examples 10 and 11 of the first embodiment.
FIG. 10 shows photographs of immunostained cells in Example 10 of the first embodiment.
FIG. 11 shows photographs of immunostained cells in Example 11 of the first embodiment.
FIG. 12 is a graph showing the average number of monocytes per 10 mL of blood in Example 12 of the first embodiment.
FIG. 13 shows flow cytometry dot-plots of the results of Example 12 of the first embodiment.
FIG. 14 shows fluorescent microscope photographs of the results of Example 13 of the first embodiment.
FIG. 15 shows flow cytometry dot-plots of the results of Example 13 of the first embodiment.
FIG. 16 shows photographs of iPS cell colonies in Example 14 of the first embodiment.
FIG. 17 shows the components of the reprogramming factor RNA master mix used in the Examples of the second embodiment.
FIG. 18 is a table showing the contents of the kit used in the Examples of the second embodiment.
FIG. 19 shows a photograph of iPS cell colonies in the Examples of the second embodiment.
FIG. 20 shows a fluorescent microscope photograph of a Reference Example of the second embodiment.
FIG. 21 shows graphs of fluorescence activated flow cytometry analysis results of the reference example of the second embodiment.

### DESCRIPTION OF EMBODIMENTS

Embodiments of the invention will now be explained in detail. The embodiments described below are merely examples of devices and methods for implementing the technical concept of the invention, and the technical concept of the invention is not limited to the described combinations of structural members. The technical concept of the invention may incorporate various modifications such as are within the scope of the claims.

### (First Embodiment)

The production method for an artificial pluripotent stem cell (iPS cell) according to the first embodiment includes preparing a somatic cell and introducing a Sendai virus comprising reprogramming factor RNA into the somatic cell.

Examples of the somatic cell include fibroblasts, blood cells, dental pulp stem cells, keratinocytes, dermal papilla cells, oral epithelial cells, and somatic stem progenitor cells.

Blood cells are separated from blood. The blood may be, but is not limited to, peripheral blood and umbilical cord blood. The blood may be harvested from an adult or from a juvenile. An anticoagulant such as ethylenediaminetetraacetic acid (EDTA), heparin or biologically standardized blood storage Solution A (ACD-A) may be used for blood harvesting.

Blood cells are, for example, nucleated cells such as monocytes, neutrophils, eosinophils, basophils and lymphocytes, and do not include erythrocytes, granulocytes or platelets. The blood cells may be vascular endothelial precursor cells, blood stem/progenitor cells, T cells or B cells. T cells may be αβ T cells, for example.

Monocytes are separated from blood using a blood cell separation medium and a centrifugal separation apparatus. The method for separating monocytes when using Ficoll (GE Healthcare) as the blood cell separation medium is as follows.

Because the separation precision for monocytes tends to be poor at low temperatures, the centrifuge is set to from 4 °C to 42 °C, and preferably to 18 °C. After collecting 10 µL to 50 mL of blood from an adult or juvenile human, a chelating agent containing EDTA is added and gently mixed into the blood to prevent the blood from coagulating. Also, a medium for human lymphocyte separation (Ficoll-Paque PREMIUM, GE Healthcare, Japan) is dispensed into two 15 mL tubes at 5 mL each. After adding 5 mL of PBS to 5 mL of the blood for dilution, 5 mL thereof is overlaid onto the human lymphocyte separation medium in each of the tubes. At this time, the diluted blood is slowly added onto the medium down the tube wall so as not to disturb the interface.

The solution in the tube is centrifuged at from 10 × g to 1000 × g, and preferably at 400 × g, for from 5 minutes to 2 hours, and preferably for 30 minutes, at from 4 °C to 42 °C, and preferably at 18 °C. After centrifugation, a white cloudy intermediate layer appears in the tube. The white cloudy intermediate layer includes monocytes. The white cloudy intermediate layer in each tube is slowly collected with a Pipetman and transferred to a new 15 mL tube while ensuring the lower layer is not sucked up. Approximately 1 mL of the white cloudy intermediate layer can be collected from each tube. The intermediate layers of two tubes are combined and transferred to a single tube.

After adding 1 mL to 48 mL, and preferably 12 mL of PBS to the collected monocytes, the solution is further centrifuged at from 10 × g to 1000 × g, and preferably at 200 × g, at from 4 °C to 42 °C, and preferably at 18 °C, for from 1 minute to 60 minutes, and preferably for 10 minutes. Next, an aspirator is used to draw out and remove the supernatant of the solution, and 1 mL to 12 mL, and preferably 3 mL of a serum-free hematopoietic cell medium of known composition (X-VIVO® 10, Lonza) is added to obtain a monocyte suspension. A 10 µL portion of the monocyte suspension is stained with Trypan blue and the count is determined with a hemocytometer.

The method for separating the monocytes when using a Vacutainer® (BD) as a blood sampling tube is as follows.

Because the separation precision for monocytes tends to be poor at low temperatures, the centrifuge is set to from 4 °C to 42 °C, and preferably to 18 °C. A blood sampling tube (Vacutainer®, BD) is used to harvest 8 mL of blood from an adult or juvenile human, and inverting mixing is carried out for mixing with an anticoagulant. The balance is then adjusted, and the solution is centrifuged with a swing rotor at from 4 °C to 42 °C, and preferably at 18 °C, at from 100 × g to 3000 × g, and preferably from 1500 × g to 1800 × g, for from 1 minute to 60 minutes, and preferably for 20 minutes. After centrifugation, the upper layer (blood plasma layer) is removed, and pipetting is performed to obtain the mononuclear cell layer and a suspension in which the gel-adhering blood cells are suspended. The obtained suspension is transferred to a separate 15 mL tube.

After adding 1 mL to 14 mL, and preferably 12 mL of PBS to the suspension in the 15 mL tube, the suspension is centrifuged at from 4 °C to 42 °C, and preferably at 18 °C, at from 100 × g to 3000 × g, and preferably at 200 × g, for from 1 minute to 60 minutes, and preferably for 5 minutes. After centrifugation, the supernatant is removed with an aspirator. A hemolytic agent (PharmLyse®, 10-fold concentration, BD) is diluted to 1-fold concentration with sterilized water. The pellet in the 15 mL tube is broken up by tapping, and 1 mL and 14 mL, and preferably 1 mL of hemolytic agent is added. It is then shielded from light at room temperature, and the solution is allowed to stand for from 1 minute to 60 minutes, and preferably for 1 minute.

After then adding 1 mL to 14 mL, and preferably 12 mL of PBS to the 15 mL tube, it is centrifuged at from 4 °C to 42 °C, and preferably at room temperature, at from 100 × g to 3000 × g, and preferably at 200 × g, for from 1 minute to 60 minutes, and preferably for 5 minutes. After centrifugation, an aspirator is used to remove the supernatant, and 1 mL and 15 mL, and preferably 3 mL of a serum-free hematopoietic cell medium of known composition (X-VIVO® 10, Lonza) is added to obtain a monocyte suspension. A 10 µL portion of the monocyte suspension is stained with Trypan blue and the count is determined with a hemocytometer.

The method for separating the monocytes from blood is not limited to the method described above, and the monocytes may be separated from the blood using a dialysis membrane, for example. Further, Purecell Select System® (PALL) for whole blood monocyte enrichment, a purifier (Cellsorba E®, Asahi Kasei Corp.) for removing blood cells, and filters such as a leukocyte removal filter (SEPACELL PL®, PLX-5B-SCD, Asahi Kasei Corp.) for platelet preparation may also be used.

The monocytes may be separated using an erythrocyte separating agent that is able to separate nucleated cells by gravity sedimentation or centrifugal separation of erythrocytes. Examples of erythrocyte separating agents include HetaSep® (Stemcell Technologies) and HES40 (Nipro).

The monocytes used may be CTL-UP1, available from Cellular Technology Limited, or PBMC-001 from Sanguine Biosciences.

Alternatively, the blood cells may be blood cells that have been cryopreserved using a cell cryopreservation liquid such as CELLBANKER 1, STEMCELLBANKER GMP grade, or STEMCELLBANKER DMSO-free GMP grade (Zenoaq), and then thawed.

For thawing of the monocytes, first from 1 mL to 15 mL, and preferably 8 mL of serum-free hematopoietic cell medium of known composition (X-VIVO® 10, Lonza) is placed in a 15 mL tube, and the tube containing the frozen monocytes is set in a hot bath at from 4 °C to 42 °C and preferably at 37 °C to begin to thaw the monocytes. Next, while some of the ice is remaining, the tube containing the monocytes is pulled out from the hot bath and transferred to a tube containing serum-free hematopoietic cell medium of known composition. A 10 µL portion of the monocyte suspension is stained with Trypan blue and the count is determined with a hemocytometer.

The blood cells may be separated based on the presence of a cell surface marker. Blood stem/progenitor cells are CD34-positive. T cells are positive for CD3, CD4 or CD8. B cells are positive for CD10, CD19 or CD20. Blood stem/progenitor cells, T cells, or B cells are separated from blood cells using an automatic magnetic cell separator and immunomagnetic beads, for example. Alternatively, pre-separated monocytes may be prepared. However, blood cells that have not been separated based on the presence of a cell surface marker may also be used.

CD34-positive cells are stem cells or progenitor cells and tend to be easily reprogrammable. When iPS cells are prepared using T cells, which are CD3-positive cells, the T cell-derived iPS cells retain their TCR recombination form, so that it is generally possible to efficiently induce differentiation to T cells.

The method for separating CD34-positive cells is as follows.

A blood cell medium (blood stem/progenitor cell medium) is prepared by adding 10 µL of IL-6 (100 µg/mL), 10 µL of SCF (300 µg/mL), 10 µL of TPO (300 µg/mL), 10 µL of FLT3 ligand (300 µg/mL) and 10 µL of IL-3 (10 µg/mL) to 10 mL of serum-free medium (StemSpan H3000, Stemcell Technologies).

From 1 mL to 6 mL, and preferably 2 mL of the blood cell medium is loaded into one well of a 6-well plate. In order to prevent evaporation of the medium, 1 mL to 6 mL or 2 mL of PBS is loaded into each of the other 5 wells. The 6-well plate is then placed in an incubator at from 4 °C and 42 °C, and preferably at 37 °C, and incubated.

A column buffer is prepared with from 10 µL to 1 mL, and preferably 80 µL of EDTA (500 mmol/L) and from 10 µL to 1 mL, and preferably 200 µL of FBS, added to 20 mL of PBS. A monocyte suspension containing from 1 × 10⁴ to 1 × 10⁹, and preferably 2 × 10⁷ monocytes is dispensed into a 15 mL tube, and the monocyte suspension is centrifuged for 10 minutes at from 4 °C and 42 °C, and preferably at 4 °C, at from 100 × g to 3000 × g, and preferably at 300 × g. After centrifugation, the supernatant is removed and the monocytes are suspended in from 100 µL to 1 mL, and preferably in 300 µL/ of column buffer.

Next, 10 µL to 1 mL, and preferably 100 µL of FcR blocking reagent (Miltenyi Biotec) and 10 µL and 1 mL, and preferably 100 µL of a CD34 microbead kit (Miltenyi Biotec) are added to the monocyte suspension in the 15 mL tube. FcR blocking reagent is used to increase the microbead-labeling specificity. The monocyte suspension is then mixed in, and the mixture is allowed to stand at from 4 °C to 42 °C, and preferably at 4 °C, for from 1 minute to 2 hours, and preferably for 30 minutes.

Next, 1 mL to 15 mL, and preferably 10 mL of column buffer is added to the monocyte suspension in the 15 mL tube for dilution, and the mixture is centrifuged at from 4 °C to 42 °C, and preferably at 4 °C, at from 100 × g to 1000 × g, and preferably at 300 × g, for from 1 minute to 2 hours, and preferably for 10 minutes. After centrifugation, the supernatant in the 15 mL tube is removed with an aspirator, and 10 µL to 10 mL, and preferably 500 µL of column buffer is added for resuspension.

An automatic magnetic cell separator column (MS column, Miltenyi Biotec) is mounted in an automatic magnetic cell separator (MiniMACS Separation Unit, Miltenyi Biotec), and 10 µL to 10 mL, and preferably 500 µL of column buffer is loaded into the column and rinsing is carried out. The monocytes are then loaded into the column. After then loading 10 µL to 10 mL, and preferably 500 µL of column buffer into the column, the column is rinsed 1 to 10 times, and preferably 3 times. The column is then removed from the automatic magnetic cell separator and placed in a 15 mL tube. Next, 10 µL to 10 mL, and preferably 1000 µL of column buffer is loaded into the column and a syringe is rapidly pressed in to discharge the CD34-positive cells into the 15 mL tube.

A 10 µL portion of the CD34-positive cell suspension is dyed with Trypan blue, and the cell count is determined using a blood cell counting chamber. The CD34-positive cell suspension in the 15 mL tube is centrifuged at from 4 °C to 42 °C, and preferably at 4 °C, at from 100 × g to 1000 × g, and preferably at 300 × g, for from 1 minute to 2 hours, and preferably for 10 minutes. After centrifugation, the supernatant is removed with an aspirator. The CD34-positive cells are resuspended in preheated blood cell medium, and the CD34-positive cells are spread onto a culture plate. The CD34-positive cells are then cultured for 6 days at from 4 °C to 42 °C, and preferably at 37 °C, with from 1% to 20%, and preferably with 5% CO₂. There is no need for medium exchange during this procedure.

The method for isolating cells with a marker other than CD34 is the same as the method for isolating CD34-positive cells.

Sendai virus comprising reprogramming factor RNA is introduced into adhesion-cultured somatic cells. Alternatively, Sendai virus comprising reprogramming factor RNA is introduced into somatic cells suspension cultured in a gel culture medium.

Somatic cells into which reprogramming factor RNA will be introduced are cultured feeder-free using a basal membrane matrix such as Matrigel (Corning), CELLstart® (ThermoFisher), or Laminin511 (iMatrix-511, Nippi).

Stem cell culture media such as human ES/iPS culture media e.g. Primate ES Cell Medium (ReproCELL) can be used for the culture medium in which somatic cells into which reprogramming factor RNA will be introduced are cultured.

However, the stem cell culture medium is not limited thereto and various stem cell culture media can be used. For example, Primate ES Cell Medium, Reprostem, ReproFF, ReproFF2, ReproXF (Reprocell), mTeSR1, TeSR2, TeSRE8, ReproTeSR (STEMCELL Technologies), PluriSTEM® Human ES/iPS Medium (Merck), NutriStem® XF/FF Culture Medium for Human iPS and ES cells, Pluriton reprogramming medium (Stemgent), PluriSTEM® Stemfit AK02N, Stemfit AK03 (Ajinomoto), ESC-Sure® serum and feeder-free medium for hESC/iPS (Applied StemCell), L7® hPSC Culture System (LONZA), and PluriQ (MTI-GlobalStem) can be used. The stem cell culture medium may be added to dishes, wells, tubes, etc.

A gel culture medium is prepared by adding, for example, gellan gum to the stem cell culture medium so the final concentration thereof is 0.001% by mass to 0.5% by mass, 0.005% by mass to 0.1% by mass, or 0.01% by mass to 0.05% by mass.

The gel culture medium may comprise at least one high molecular compound selected from the group consisting of gellan gum, hyaluronic acid, rhamsan gum, diutan gum, xanthan gum, carrageenan, fucoidan, pectin, pectic acid, pectinic acid, heparan sulfate, heparin, heparitin sulfate, kerato sulfate, chondroitin sulfate, dermatan sulfate, rhamnan sulfate, and salts thereof. Further, the gel culture medium may comprise methyl cellulose. By including methyl cellulose, aggregation of cells is further suppressed.

Alternatively, the gel culture medium may comprise at least one temperature sensitive gel selected from the group consisting of poly(glycerol monomethacrylate)(PGMA), poly(2-hydroxypropyl methacrylate)(PHPMA), poly(N-isopropyl acrylamide)(PNIPAM), amine terminated, carboxylic acid terminated, maleimide terminated, N-hydroxysuccinimide(NHS)ester terminated, triethoxysilane terminated, poly(N-isopropylacrylamide-co-acrylate), poly(N-isopropylacrylamide-co-acrylic acid), poly(N-isopropylacrylamide-co-butylacrylate), poly(N-isopropylacrylamide-co-methacrylic acid), poly(N-isopropylacrylamide-co-methacrylic acid-co-octadecyl acrylate), and N-isopropylacrylamide.

The gel culture medium does not include a growth factor such as basic fibroblast growth factor (bFGF). Alternatively, the gel culture medium may include a growth factor such as bFGF at a low concentration of 400 µg/L or less, 40 µg/L or less, or 10 µg/L or less.

Further, the gel culture medium does not include TGF-β or includes TGF-β at a low concentration of 600 ng/L or less, 300 ng/L or less, or 100 ng/L or less.

The gel culture medium need not be stirred and need not contain a feeder cell.

The gel culture medium may comprise at least one substance selected from the group consisting of cadherin, laminin, fibronectin, and vitronectin.

The reprogramming factor RNA introduced into somatic cells includes, for example, OCT3/4 mRNA, SOX2 mRNA, KLF4 mRNA, and c-MYC mRNA. M₃O, which is improved OCT3/4, may be used as the reprogramming factor. Furthermore, the reprogramming factor RNA may further include at least one mRNA factor selected from the group consisting of LIN28A, FOXH1, LIN28B, GLIS1, p53-dominant negative, p53-P275S, L-MYC, NANOG, DPPA2, DPPA4, DPPA5, ZIC3, BCL-2, E-RAS, TPT1, SALL2, NAC1, DAX1, TERT, ZNF206 , FOXD3, REX1, UTF1, KLF2, KLF5, ESRRB, miR-291-3p, miR-294, miR-295, NR5A1, NR5A2, TBX3, MBD3sh, TH2A, TH2B, and P53 DD. These mRNA are available from TriLink. Note that the gene symbols used herein refer to human genes but there is no intention to restrict the species by the use of uppercase or lowercase symbols. For example, even if all of the symbols are uppercase, this is not intended to exclude genes of mice or rats. In the Examples, however, the gene symbols are given according to the actual biological species used.

The mRNA may be modified with one or more selected from the group consisting of pseudouridine (Ψ), 5-methyluridine (5meU), N1-methylpseudouridine (me1Ψ), 5-methoxyuridine (5moU), 5-hydroxymethyluridine (5hmU), 5-formyluridine (5fU), 5-carboxymethyl esteruridine (5cam U), thienoguanosine (thG), N4-methylcytidine (me4C), 5-methylcytidine (m5C), 5-methyoxycytidine (5moC), 5-hydroxymethylcytidine (5hmC), 5-hydroxycytidine (5hoC), 5-formcytidine (5fC), 5-carboxycytidine (5caC), N6-methyl-2-aminoadenosine (m6DAP), diaminopurine (DAP), 5-methyluridine (m5U), 2'-O-methyluridine (Um or m2'-OU), 2-thiouridine (s2U), and N6-methyl adenosine (m6A).

The mRNA may also be polyadenylated.

The mRNA may be prepared by polyadenylation of (IVT) RNA that is transcribed *in vitro.* The mRNA may also be polyadenylated during IVT using a DNA template coding for poly (A) ends. The mRNA may also be capped. Most of the mRNA molecules are preferably given caps to maximize expression efficiency in the cells. The mRNA may also have a 5'cap[m7G(5')ppp(5')G] structure. This sequence stabilizes mRNAs and promotes transcription. In the case of mRNA containing 5'-triphosphate, the 5'-triphosphate may be removed therefrom by dephosphorylation treatment. The mRNA may also have [3'O-Me-m7G(5')ppp(5')G] as an Anti-Reverse Cap Analog (ARCA). ARCA is a sequence inserted before the transcription initiation site that doubles the efficiency of the mRNA to be transcribed. The mRNA may also have a poly-A tail.

The mRNA may also be replicative RNA with the ability to self-replicate. Replicative RNA is RNA with the ability to self-replicate and differs from ordinary RNA in that replicative RNA has the ability to express proteins necessary for replication of RNA. Replicative RNA is derived from Venezuelan Equine Encephalitis (VEE) virus, a type of alpha virus. Transfecting cells with replicative RNA allows the cells to express RNA that will continue to produce the reprogramming factor, thus making it possible to eliminate repeated introduction of reprogramming factor RNA into the cells.

The replicative RNA sequence may include a sequence obtained from an alpha virus selected from the group consisting of alpha virus replicon RNA, Eastern Equine Encephalitis virus (EEE), Venezuelan Equine Encephalitis virus (VEE), Everglades virus, Mucambo virus, Pixuna virus, and Western Equine Encephalitis virus (WEE).

The replicative RNA may also include a sequence obtained from an alpha virus selected from the group consisting of Sindbis virus, Semliki Forest virus, Middelburg virus, Chikungunya virus, O'nyong-nyong virus, Ross River virus, Barmah Forest virus, Getah virus, Sagiyama virus, Bebaru virus, Mayaro virus, Una virus, Aura virus, Whataroa virus, Babanki virus, Kyzylagach virus, Highlands J virus, Fort Morgan virus, Ndumu virus and Buggy Creek virus.

The replicative RNA includes, from the 5' end to the 3' end, (VEE RNA replicase)-(promoter)-(RFl)-(self-cleaving peptide)-(RF2)-(self-cleaving peptide)-(RF3)-(IRES or core promoter)-(RF4)-(IRES or arbitrary promoter)-(arbitrary selectable marker)-(VEE 3'UTR and poly-A tail)-(arbitrary selectable marker)-promoter, for example. The RF1-4 mentioned above is a factor that induces dedifferentiation of cells to pluripotent cells. The RF2-3, RF3-4 and RF4 mentioned above are optional. The RF1-4 may be selected from the group consisting of OCT3/4, KLF4, SOX-2, c-MYC, LIN28A, LIN28B, GLIS1, FOXH1, p53-dominant negative, p53-P275S, L-MYC, NANOG, DPPA2, DPPA4, DPPA5, ZIC3, BCL-2, E-RAS, TPT1, SALL2, NAC1, DAX1, TERT, ZNF206, FOXD3, REX1, UTF1, KLF2, KLF5, ESRRB, miR-291-3p, miR-294, miR-295, NR5A1, NR5A2, TBX3, MBD3sh, TH2A and TH2B.

The reprogramming factor RNA is introduced into somatic cells using Sendai virus. CytoTune® (Invitrogen) may be used for the Sendai virus loaded with reprogramming factor RNA. Multiplicity of infection (MOI) may be used as an indication of the titer of the Sendai virus. The MOI of the Sendai virus is, for example, 0.1 to 100.0, or 1.0 to 50.0.

Sendai virus is used to introduce reprogramming factor RNA to somatic cells, thereafter the cells are reprogrammed in liquid culture medium that is not gel culture medium or in a gel culture medium.

Whether or not the somatic cells have been induced (reprogrammed) into artificial pluripotent stem cells can be confirmed, for example, from the morphology of the cells. Namely, whether or not somatic cells have been induced into artificial pluripotent stem cells can be determined by analyzing whether the cells are positive for at least one cell surface marker selected from TRA-1-60, TRA-1-81, SSEA-1, and SSEA5, which are cell surface markers indicating the cells are undifferentiated, using a flow cytometer. TRA-1-60 is a specific antigen for iPS/ES cells which cannot be detected on somatic cells. Since iPS cells can only have been made in the TRA-1-60 positive fraction, TRA-1-60 positive cells are considered to be a type of iPS cell.

According to the production method for an artificial pluripotent stem cell of the first embodiment described above, RNA that can express reprogramming factor RNA is efficiently introduced into somatic cells, the reprogramming factor RNA is expressed, and artificial pluripotent stem cells can be produced.

### (Example 1 of the first embodiment)

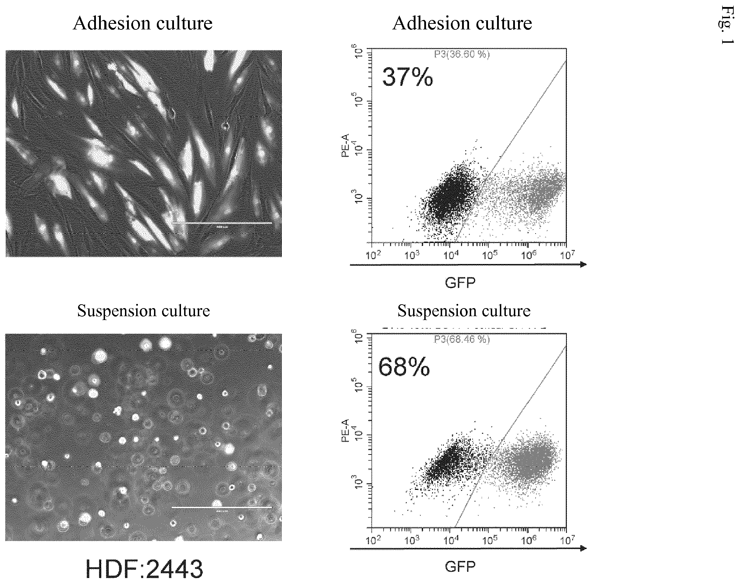

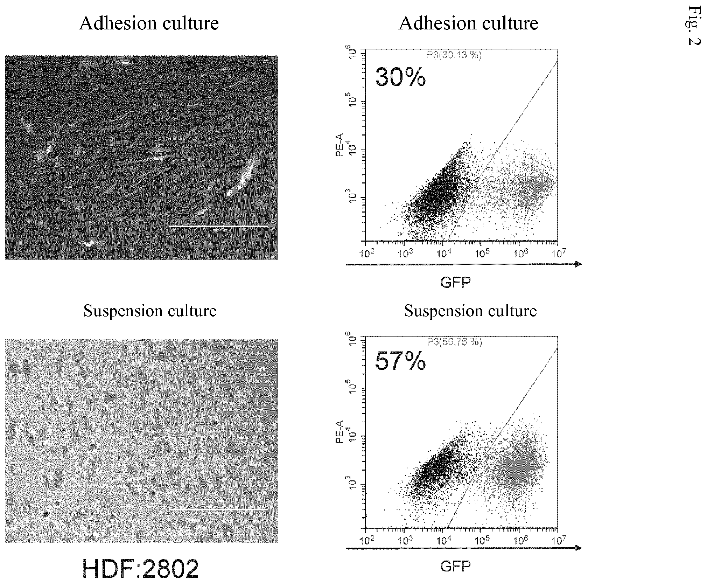

1 × 10⁵ fibroblasts (HDF2443 or HDF2802, Cell Applications, Inc) suspended in 2 mL of 10% FBS- (Gibco) containing DMEM (Gibco) used as a liquid culture medium that is not a gel culture medium was seeded in each of the wells of a well dish, the well dish was left standing in a CO₂ incubator at 37 °C, and the fibroblasts were adhesion-cultured. 24 hours later, a Sendai virus (CytoTune®, EmGFP Sendai Fluorescence Reporter, Invitorogen) was added to the liquid culture medium at a multiplicity of infection (MOI) of 3.0. 24 hours later, a fluorescence microscope was used to observe the fluorescence intensity of the fibroblasts as shown in FIG. 1 (HDF2443) and FIG. 2 (HDF2802). Further, a flow cytometer was used to measure the fluorescence intensity. The results confirmed that the fibroblasts were infected with Sendai virus.

### (Example 2 of the first embodiment)

1 × 10⁵ fibroblasts (HDF2443 or HDF2802) were suspended in a 2 mL gel culture medium of 10% FBS-containing DMEM (Gibco) to which Gellan gum (Nissan Chemical) was added so as to be 0.02% by mass, and a tube was seeded therewith. The tube was left standing in a CO₂ incubator at 37 °C and the fibroblasts were suspension cultured. 24 hours later, a Sendai virus that can cause the expression of the fluorescence protein EGFP (CytoTune®, EmGFP Sendai Fluorescence Reporter, Invitorogen) was added to the gel culture medium at an MOI of 3.0. 24 hours later, a fluorescence microscope was used to observe the fluorescence intensity of the fibroblasts as shown in FIG. 1 (HDF2443) and FIG. 2 (HDF2802). Further, a flow cytometer was used to measure the fluorescence intensity. The results confirmed that the fibroblasts were infected with Sendai virus.

Furthermore, from the results of Examples 1 and 2 of the first embodiment, more fluorescence protein EGFP was expressed when suspension culturing was performed in the gel culture medium than when adhesion culturing was performed in the liquid culture medium. This shows that the Sendai virus was more efficiently introduced into the cells when suspension culturing in a gel culture medium than when adhesion culturing in a liquid culture medium.

### (Example 3 of the first embodiment)

1 × 10⁵ fibroblasts (HDF1419 Cell Applications, Inc.) were suspended in a 2 mL gel culture medium of 4 ng/mL of bFGF- (Gibco) containing hES culture medium (Primate ES Cell Medium, ReproCELL Inc.) to which gellan gum (Nissan Chemical) was added so as to be 0.02% by mass, and a tube was seeded therewith. The tube was left standing in a CO₂ incubator at 37 °C and the fibroblasts were suspension cultured. 24 hours later, a Sendai virus (Invitorogen) that can express reprogramming factors OSKM (OCT3/4, SOX, KLF4, c-MYC) was added to the gel culture medium. The Sendai virus was added to the gel culture medium such that the hKOS (KLF4, OCT3/4, SOX2) MOI was 5.0, the c-MYC MOI was 5.0, and the hKLF MOI was 5.0.

Thereafter, the tube was left standing in a CO₂ incubator at 37 °C. The cells were cultured continuously for 23 days. During that time, 500 µl of bFGF-containing gel culture medium was added to the tube every 48 hours.

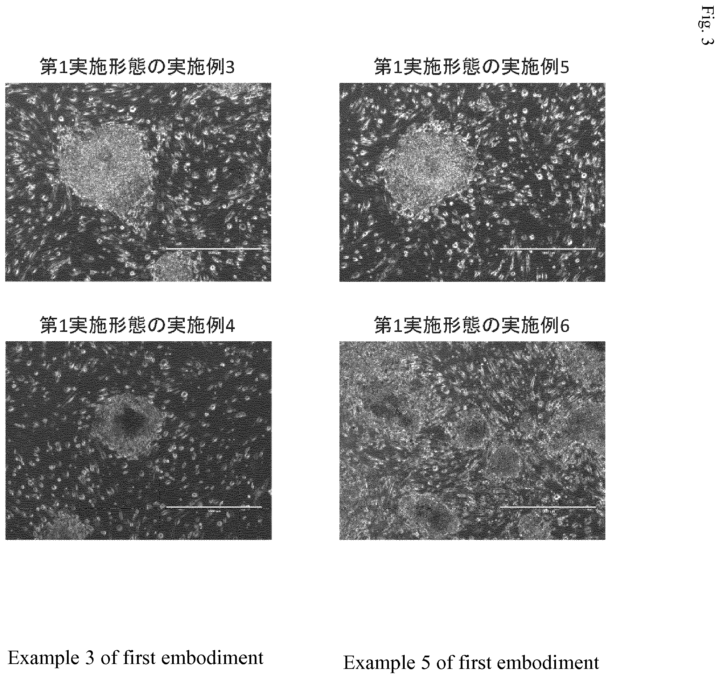

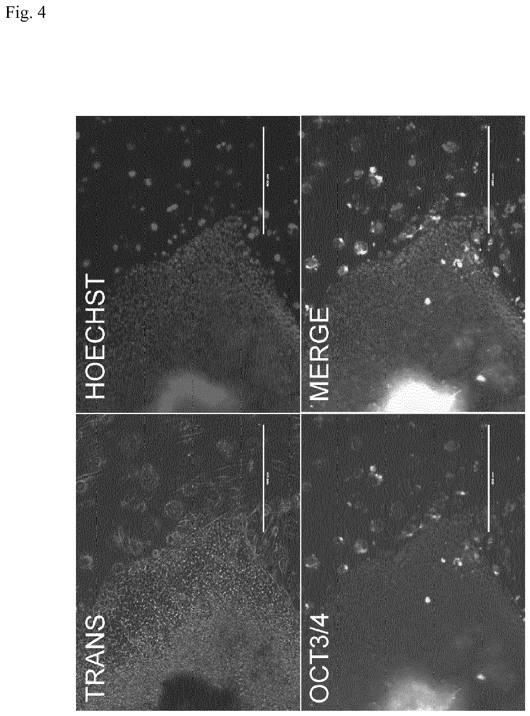

The results confirmed the formation of iPS cell clumps in the bFGF-containing gel culture medium. Further, iPS cells produced in a bFGF-containing gel culture medium were reseeded onto a feeder and the morphology of the colonies was confirmed. As can be seen in FIG. 3, the morphology was that of undifferentiated iPS cell colonies. Furthermore, immunostaining the cells with antibodies against OCT3/4 which is a marker for pluripotent stem cells showed that the cells were OCT3/4 positive as shown in FIG. 4.

### (Example 4 of the first embodiment)

Other than adding Sendai virus to a gel culture medium such that the MOI of hKOS (KLF4, OCT3/4, SOX2) was 2.5, the MOI of c-MYC was 2.5, and the MOI of hKLF was 2.5, the fibroblasts (HDF1419, Cell Applications, Inc.) were reprogrammed in the same way as in Example 3 of the first embodiment.

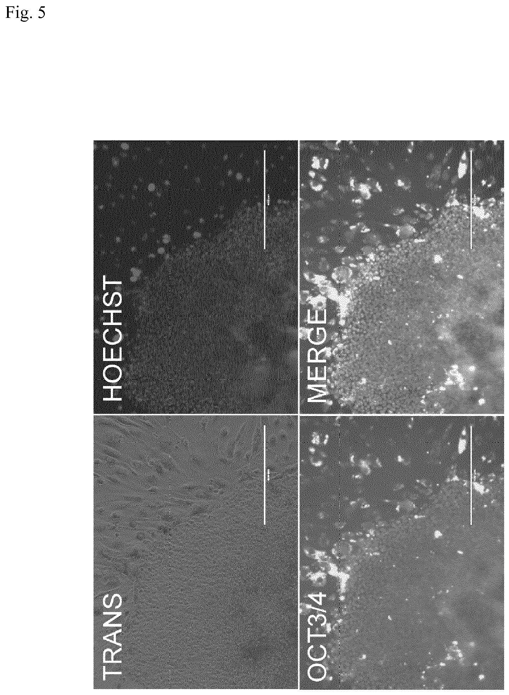

The results confirmed the formation of iPS cell clumps in the bFGF-containing gel culture medium. Further, iPS cells produced in bFGF-containing gel culture medium were reseeded onto a feeder and the morphology of the colonies was confirmed. As can be seen in FIG. 3 the morphology was that of undifferentiated iPS cell colonies. Furthermore, immunostaining the cells with antibodies against OCT3/4 which is a marker for pluripotent stem cells showed that the cells were OCT3/4 positive as shown in FIG. 5.

### (Example 5 of the first embodiment)

Other than using bFGF-containing gel culture medium, the fibroblasts (HDF1419, Cell Applications, Inc.) were reprogrammed in the same way as in Example 3 of the first embodiment.

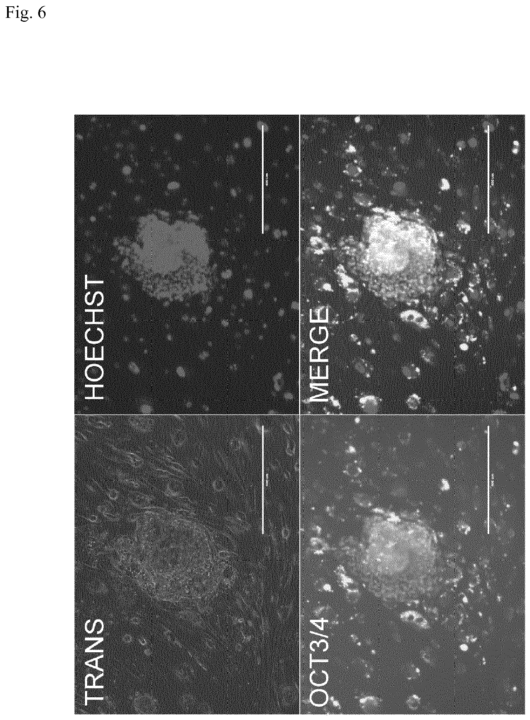

The results confirmed the formation of iPS cell clumps in non-bFGF-containing gel culture medium. Further, iPS cells produced in a non-bFGF-containing gel culture medium were reseeded onto a feeder and the morphology of the colonies was confirmed. As can be seen in FIG. 3 the morphology was that of undifferentiated iPS cell colonies. Furthermore, immunostaining the cells with antibodies against OCT3/4 which is a marker for pluripotent stem cells showed that the cells were OCT3/4 positive as shown in FIG. 6.

### (Example 6 of the first embodiment)

Other than using fibroblasts (HDF2802, Cell Applications, Inc), the fibroblasts (HDF2802, Cell Applications, Inc.) were reprogrammed in the same way as in Example 5 of the first embodiment.

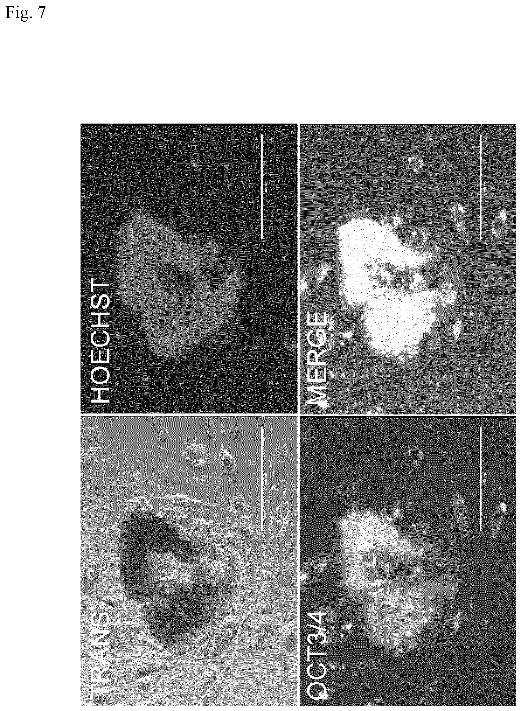

The results confirmed the formation of iPS cell clumps in non-bFGF-containing gel culture medium. Further, iPS cells produced in non-bFGF-containing gel culture medium were reseeded onto a feeder and the morphology of the colonies was confirmed. As can be seen in FIG. 3 the morphology was that of undifferentiated iPS cell colonies. Furthermore, immunostaining the cells with antibodies against OCT3/4 which is a marker for pluripotent stem cells showed that the cells were OCT3/4 positive as shown in FIG. 7.

The results of Examples 3 to 6 of the first embodiment show that iPS cells could be induced from a plurality of types of fibroblast in a bFGF-containing gel culture medium and a non-bFGF-containing gel culture medium using Sendai virus.

### (Example 7 of the first embodiment)

5 × 10⁴ blood cells (monocytes, Stanford University Blood Center) were suspended in 750 µL of Stemspan ACF (Stemcell Technologies) used as a liquid culture medium that is not a gel culture medium and each of the wells of a well dish were seeded therewith, the well dish was left standing in a CO₂ incubator at 37 °C, and the blood cells were cultured. Note that 20 ng/mL of FLT 3, 10 ng/mL of TPO, 50 ng/mL of IL6, 10 ng/mL of GCSF, 50 ng/mL of SCF, and 20 ng/mL of IL3 were added as growth factors to the Stemspan ACF. The same was also performed for the following Examples using Stemspan ACF.

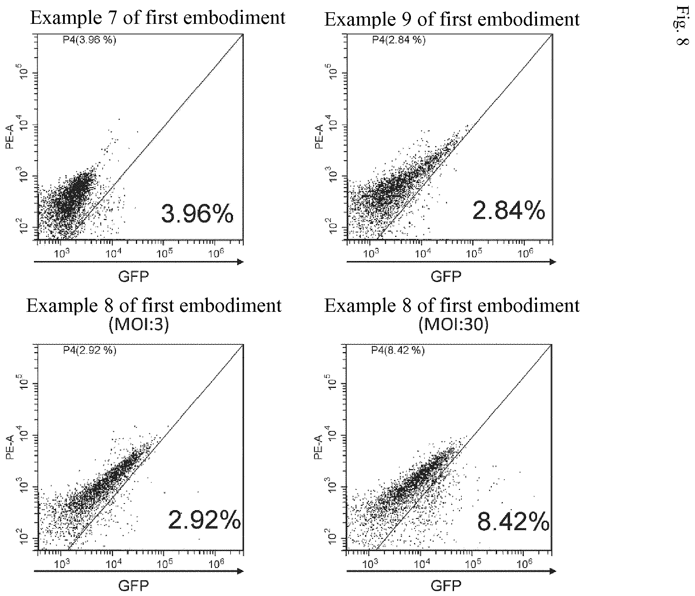

24 hours later, Sendai virus (CytoTune®, EmGFO Sendai Fluorescence Reporter, Invitorogen) that can cause the expression of fluorescence protein EGFP was added to the liquid culture medium such that the MOI thereof would be 3.0. 24 hours later a fluorescence microscope was used to observe the fluorescence intensity of the blood cells. Further, a flow cytometer was used to measure the fluorescence intensity as shown in FIG. 8. The results confirmed that the blood cells were infected with Sendai virus.

### (Example 8 of the first embodiment)

5 × 10⁴ blood cells (monocytes, Stanford University Blood Center) were suspended in 750 µl of a gel culture medium comprising Stemspan ACF (Stemcell Technologies) to which a deacylated gellan gum (Nissan Chemical) was added so as to be 0.02% by mass, and a tube was seeded therewith, the tube was left standing in a CO₂ incubator at 37 °C, and the blood cells were cultured.

24 hours later, Sendai virus (CytoTune®, EmGFO Sendai Fluorescence Reporter, Invitorogen) that can cause the expression of fluorescence protein EGFP was added to the gel culture medium such that the MOI thereof would be 3.0 or 30.0. 24 hours later a fluorescence microscope was used to observe the fluorescence intensity of the blood cells. Further, a flow cytometer was used to measure the fluorescence intensity as shown in FIG. 8. The results confirmed that the blood cells were infected with Sendai virus. Further, it was confirmed that a higher MOI allows the introduction of Sendai virus into cells more efficiently.

### (Example 9 of the first embodiment)

5 × 10⁴ blood cells (monocytes, Stanford University Blood Center) were suspended in 100 µL of a gel culture medium comprising Stemspan ACF (Stemcell Technologies) to which a gellan gum (Nissan Chemical) was added so as to be 0.02% by mass, and a tube was seeded therewith. The tube was left standing in a CO₂ incubator at 37 °C, and the blood cells were suspension cultured.

24 hours later, Sendai virus (CytoTune®, EmGFO Sendai Fluorescence Reporter, Invitorogen) that can cause the expression of fluorescence protein EGFP was added to the gel culture medium such that the MOI thereof would be 30.0. 24 hours later a fluorescence microscope was used to observe the fluorescence intensity of the blood cells. Further, a flow cytometer was used to measure the fluorescence intensity as shown in FIG. 8. The results confirmed that the blood cells were infected with Sendai virus.

### (Example 10 of the first embodiment)

1 × 10⁶ blood cells (monocytes, Stanford University Blood Center) were suspended in 750 µl of Stemspan ACF (Stemcell Technologies) and each well of a well dish was seeded therewith. The well dish was left standing in a CO₂ incubator at 37 °C and the blood cells were adhesion-cultured. 3 days later, 750 µL of Stemspan ACF was added to each well, the well dish was left standing in the CO₂ incubator at 37 °C and the culturing of blood cells was continued.

After a further three days, the blood cells in each of the wells were recovered, the blood cells were centrifuged at 280 g using a centrifugal separation apparatus, the blood cells were suspended in 2 mL of a gel medium comprising non-bFGF-containing hES culture medium (Primate ES Cell Medium, ReproCELL Inc.) to which gellan gum was added so as to be 0.02% by mass, and the gel culture medium in which the blood cells had been suspended was loaded into a tube.

Sendai virus (Invitorogen) that can cause the expression of reprogramming factors OSKM (OCT3/4, SOX2, KLF4, c-MYC) was added to the gel culture medium. The Sendai virus was added to the gel culture medium such that the MOI of hKOS (KLF4, OCT3/4, and SOX2) was 10.0, the MOI of c-MYC was 10.0, and the MOI of hKLF was 10.0.

Thereafter, the tube was left standing in a CO₂ incubator at 37 °C. The cells were cultured continuously for 30 days. During that time, 500 µL of non-bFGF-containing gel culture medium was added to the tube every 48 hours.

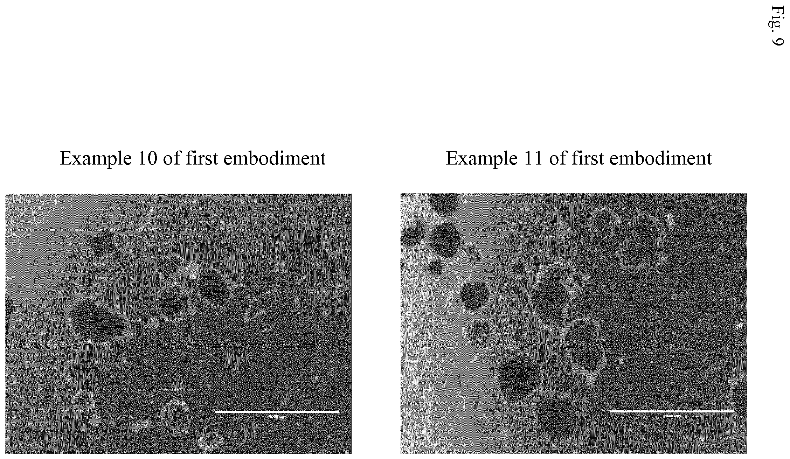

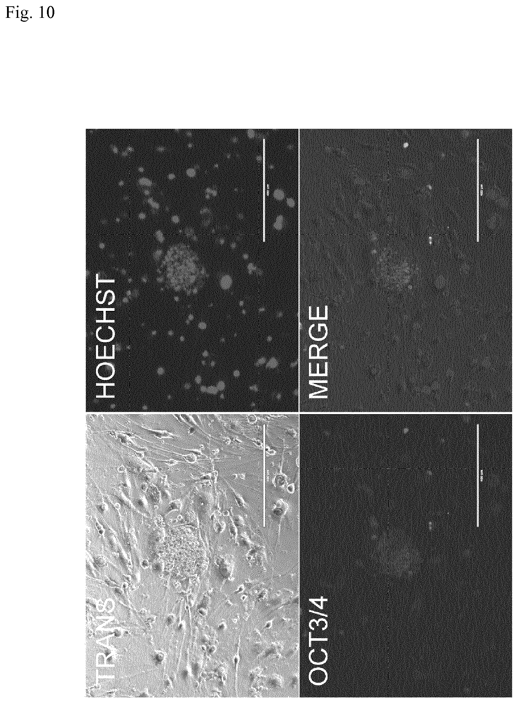

The results confirmed the formation of iPS cell clumps in non-bFGF-containing gel culture medium. Further, iPS cells produced in non-bFGF-containing gel culture medium were reseeded onto a feeder and the morphology of the colonies was confirmed. As can be seen in FIG. 9 the morphology was that of undifferentiated iPS cell colonies. Furthermore, immunostaining the cells with antibodies against OCT3/4 which is a marker for pluripotent stem cells showed that the cells were OCT3/4 positive as shown in FIG. 10.

### (Example 11 of the first embodiment)

Other than adding Sendai virus to a gel culture medium such that the MOI of hKOS (KLF4, OCT3/4, SOX2) was 5.0, the MOI of c-MYC was 5.0, and the MOI of hKLF was 2.5, the blood cells (monocytes, Stanford University Blood Center) were reprogrammed in the same way as in Example 10 of the first Embodiment.

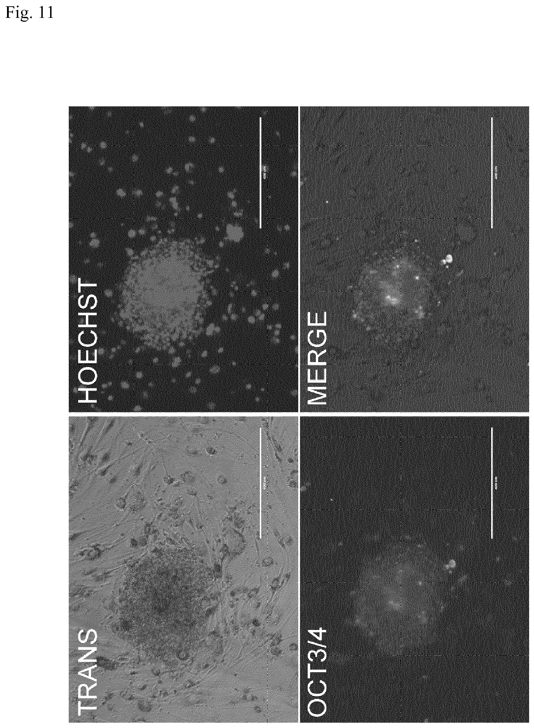

The results confirmed the formation of iPS cell clumps in non-bFGF-containing gel culture medium. Further, iPS cells produced in non-bFGF-containing gel culture medium were reseeded onto a feeder and the morphology of the colonies was confirmed. As can be seen in FIG. 9 the morphology was that of undifferentiated iPS cell colonies. Furthermore, immunostaining the cells with antibodies against OCT3/4 which is a marker for pluripotent stem cells showed that the cells were OCT3/4 positive as shown in FIG. 11.

### (Example 12 of the first embodiment)

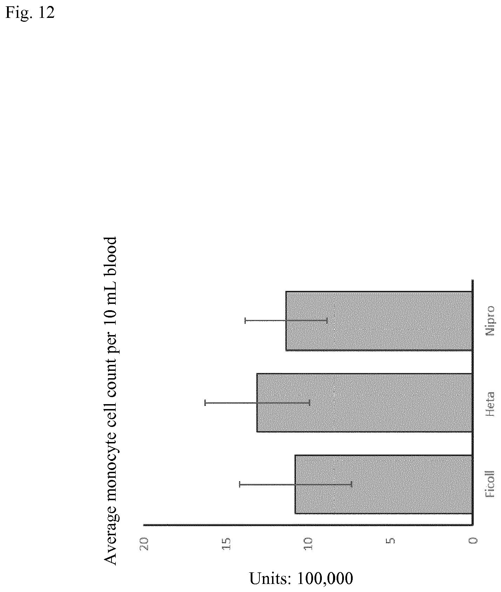

2 mL of HetaSep® (Stemcell Technologies) or HES40 (NIPRO) was added to 8 mL of whole blood, incubated in a CO₂ incubator at 37 °C for 30 minutes to 1 hour, and erythrocytes were precipitated. Thereafter, the plasma component was transferred to a tube and the number of monocytes was measured. The results are shown in FIG. 12. The monocytes could be separated at least as well as when using Ficoll (GE Healthcare).

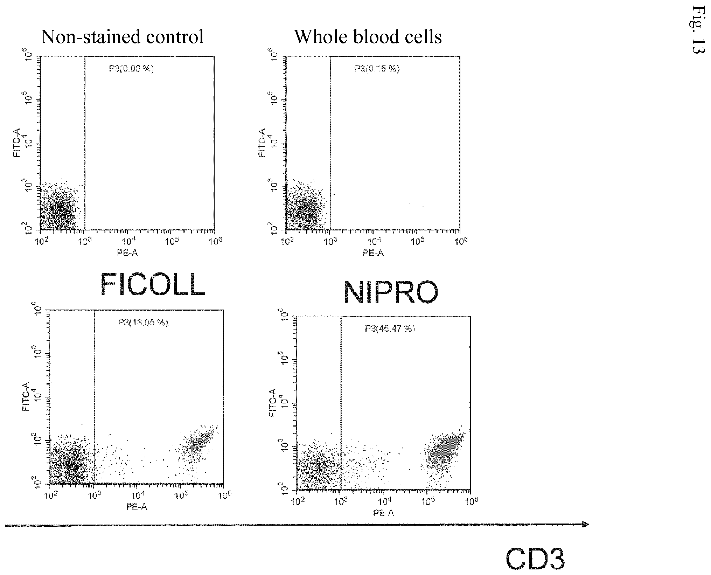

The separated monocytes were immunostained with CD3 conjugated antibodies, and the concentration of the T cell fraction was confirmed with FACS. The results are shown in FIG. 13. It was shown that monocytes could be efficiently separated using an erythrocyte separating agent.

### (Example 13 of the first embodiment)

5 × 10⁴ monocytes separated in Example 12 of the first embodiment were suspended in 750 µL of Stemspan ACF (Stemcell Technologies) used as a liquid culture medium that is not a gel culture medium. The suspension was seeded into each of the wells of a well dish, the well dish was left standing in a CO₂ incubator at 37 °C, and the monocytes were cultured.

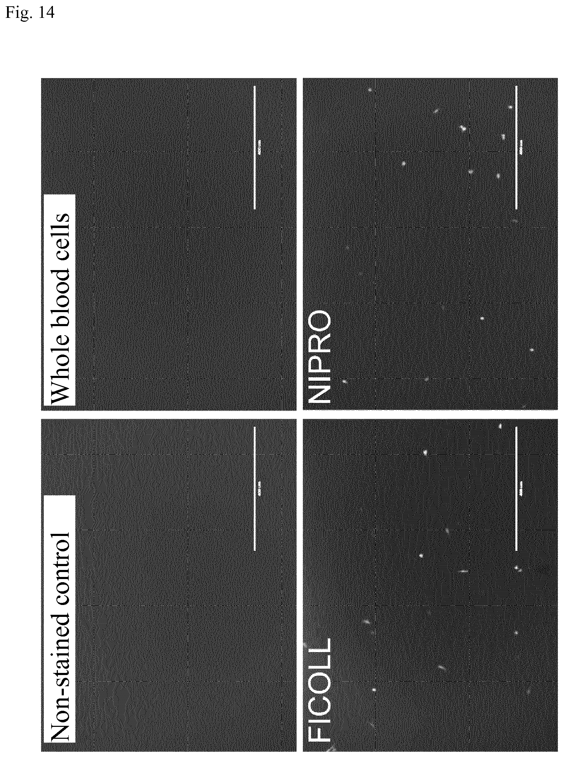

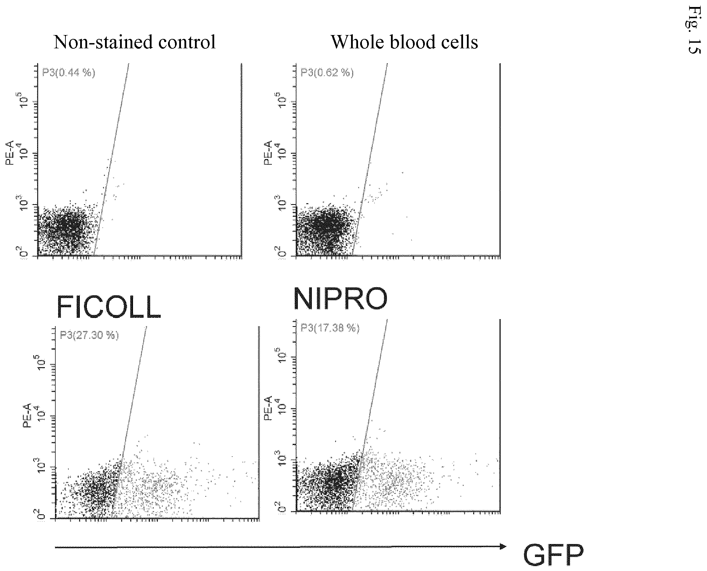

24 hours later, a Sendai virus (CytoTune®, EmGFP Sendai Fluorescence Reporter, Invitorogen) that can cause the expression of fluorescence protein EGFP was added to the liquid culture medium at an MOI of 3.0. 24 hours later a fluorescence microscope was used to observe the fluorescence intensity of the cells as shown in FIG. 14. Further, as shown in FIG. 15 a flow cytometer was used to measure the fluorescence intensity. The results confirmed that the monocytes were infected with Sendai virus.

### (Example 14 of the first embodiment)

1 × 10⁶ monocytes separated in Example 12 of the first embodiment were suspended in 750 µl of Stemspan ACF (Stemcell Technologies) used as a liquid culture medium that is not a gel culture medium. The suspension was seeded into each of the wells of a well dish, the well dish was left standing in a CO₂ incubator at 37 °C, and the monocytes were cultured. Three days later, 750 µL of Stemspan ACF was added to each well, and the well dish was left standing in the CO₂ incubator at 37 °C.

Three more days later the cells from each of the wells were collected, and 280 g of monocytes were separated using a centrifugal separation apparatus. The separated monocytes were suspended in an hES cell culture medium (Primate ES Cell Medium, ReproCELL Inc.) used as a liquid culture medium that is not a gel culture medium or non-bFGF-containing Stemspan ACF (Stemcell Technologies), and the suspension was loaded into each of the wells of the well dish.

Sendai virus (Invitorogen) that can cause the expression of reprogramming factors OSKM (OCT3/4, SOX2, KLF4, c-MYC) was added to the culture medium. The Sendai virus was added to the culture medium such that the MOI of hKOS (KLF4, OCT3/4, and SOX2) was 10.0, the MOI of c-MYC was 10.0, and the MOI of hKLF was 10.0.

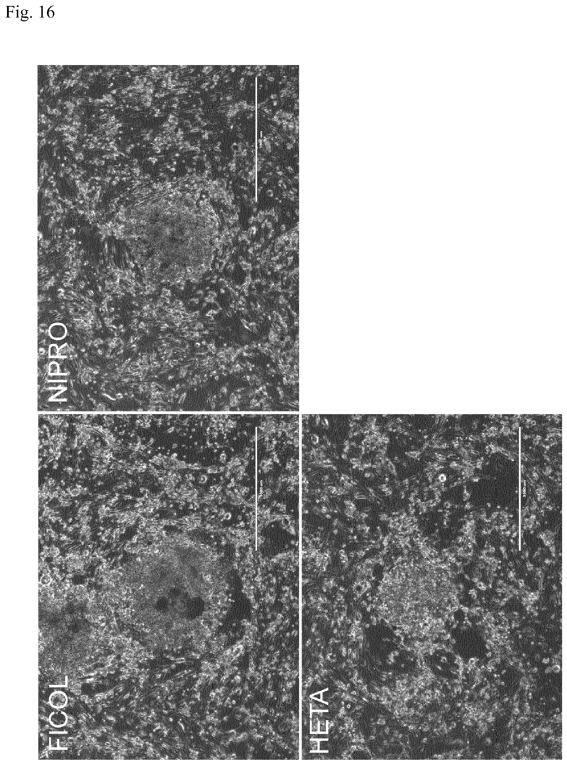

Two days after the monocytes had been infected with Sendai virus, bFGF-containing hES cell culture medium (Primate ES Cell Medium, ReproCELL Inc.) was added to the wells and a further two days later, the cells were seeded onto feeder cells. Thereafter, once the cells had been cultured on the feeder cells for 19 days, the formation of undifferentiated iPS cell colonies was confirmed as shown in FIG. 16. Thus, it was confirmed that iPS cells could be induced from monocytes separated using HetaSep® (Stem Cell Technologies) and monocytes separated using HES40 (NIPRO) in the same way as monocytes separated using Ficoll (GR Healthcare).

### (Second Embodiment)

The production method for an artificial pluripotent stem cell according to the second embodiment comprises preparing a somatic cell, introducing reprogramming factor RNA into the somatic cell using an RNA transfection agent, and reprogramming the somatic cell in a gel culture medium.

Examples of the somatic cell include fibroblasts, blood cells, dental pulp stem cells, keratinocytes, dermal papilla cells, oral epithelial cells, and somatic stem progenitor cells.

The details for the blood cells are described in the first embodiment. The details for the reprogramming factor RNA are also described in the first embodiment.

Somatic cells into which reprogramming factor RNA will be introduced are cultured feeder-free using a basal membrane matrix such as Matrigel (Corning), CELLstart® (ThermoFisher), or Laminin 511 (iMatrix-511, Nippi).

Stem cell culture media such as human ES/iPS culture media e.g. PluriQ (MTI-GlobalStem) and Primate ES Cell Medium (ReproCELL) can be used for the culture medium in which somatic cells into which reprogramming factor RNA will be introduced are cultured.

However, the stem cell culture medium is not limited thereto and various stem cell culture media may be used. For example, Primate ES Cell Medium, Reprostem, ReproFF, ReproFF2, ReproXF (Reprocell), mTeSR1, TeSR2, TeSRE8, ReproTeSR (STEMCELL Technologies), PluriSTEM® Human ES/iPS Medium (Merck), NutriStem® XF/FF Culture Medium for Human iPS and ES Cells, Pluriton reprogramming medium (Stemgent), PluriSTEM®, Stemfit AK02N, Stemfit AK03 (Ajinomoto), ESC-Sure® serum and feeder free medium for hESC/iPS (Applied StemCell), and L7® hPSC Culture System (LONZA) may be used. The stem cell medium is loaded in a dish, well or tube, for example.

The reprogramming factor RNA is introduced into somatic cells using an RNA transfection reagent. Lipofectamine MessengerMAX® may be used as the RNA transfection reagent.

Alternatively, for example, mRNA-In® (Molecular Transfer, Inc.) may be used for the RNA transfection reagent. Furthermore, the RNA transfection reagent may be a lipofection reagent such as Lipofectamine® RNAiMAX (Thermo Fisher Scientific), Lipofectamin® 2000, Lipofectamin® 3000, Neon Transfection System (Thermo Fisher Scientific), Stemfect RNA transfection reagent (Stemfect), NextFect® RNA Transfection Reagent (Bioo Scientific), Amaxa (registered product) Human T cell Nucleofector (registered product) kit (Lonza, VAPA-1002), Amaxa (registered product) Human CD34 cell Nucleofector (registered product) kit (Lonza, Inc., VAPA-1003), or ReproRNA® transfection reagent (STEMCELL Technologies).

The introduction of reprogramming factor RNA into somatic cells may be carried out a plurality of times. The introduction of reprogramming factor RNA into somatic cells is carried out once every 2 days or once a day and is repeated for 5 to 15 days, 7 to 13 days, or for 10 days. Note that if the mRNA is replicative RNA, the introduction of reprogramming factor RNA into somatic cells may be carried out only once.

After introducing reprograming factor RNA into somatic cells, the somatic cells are loaded into a gel culture medium and the somatic cells are reprogrammed.

The gel culture medium is prepared by adding gellan gum such that the final concentration thereof in the stem cell culture medium is 0.001% by mass to 0.5% by mass, 0.005% by mass to 0.1% by mass, or 0.01% by mass to 0.05% by mass

The gel culture medium may contain one or more high molecular compounds selected from the group consisting of gellan gum, deacylated gellan gum, hyaluronic acid, rhamsan gum, diutan gum, xanthan gum, carrageenan, fucoidan, pectin, pectic acid, pectinic acid, heparan sulfate, heparin, heparitin sulfate, kerato sulfate, chondroitin sulfate, dermatan sulfate, rhamnan sulfate, and salts thereof. Further, the gel culture medium may comprise methyl cellulose. By including methyl cellulose, aggregation of cells is further suppressed.

Alternatively, the gel culture medium may comprise at least one temperature sensitive gel selected from the group consisting of poly(glycerol monomethacrylate)(PGMA), poly(2-hydroxypropyl methacrylate)(PHPMA), poly(N-isopropyl acrylamide)(PNIPAM), amine terminated, carboxylic acid terminated, maleimide terminated, N-hydroxysuccinimide(NHS) ester terminated, triethoxysilane terminated, poly(N-isopropylacrylamide-co-acrylate), poly(N-isopropylacrylamide-co-acrylic acid), poly(N-isopropylacrylamide-co-butylacrylate), poly(N-isopropylacrylamide-co-methacrylic acid), poly(N-isopropylacrylamide-co-methacrylic acid-co-octadecyl acrylate), and N-isopropylacrylamide.

The gel culture medium does not include a growth factor such as basic fibroblast growth factor (bFGF). Alternatively, the gel culture medium may include a growth factor such as bFGF at a low concentration of 400 µg/L or less, 40 µg/L or less, or 10 µg/L or less.

Further, the gel culture medium does not include TGF-β or includes TGF-β at a low concentration of 600 ng/L or less, 300 ng/L or less, or 100 ng/L or less.

The gel culture medium need not be stirred and need not contain a feeder cell.

The gel culture medium may comprise at least one substance selected from the group consisting of cadherin, laminin, fibronectin, and vitronectin.

Whether or not the somatic cells were induced (reprogrammed) into becoming artificial pluripotent stem cells can be confirmed by the same method described in the first embodiment.

According to the artificial pluripotent stem cell production method of the second embodiment described above, RNA that can express reprogramming factors is efficiently introduced into somatic cells, the somatic cells are efficiently reprogrammed in the gel culture medium, and artificial pluripotent stem cells can be produced.

### (Examples of the second embodiment)

A diluted solution of a basal membrane matrix in which iMatrix-511 (Nippi) was diluted so as to be 0.5 µg/cm² with respect to 1.5 mL of PBS was added to each well. The well plate was then placed in an incubator at 37°C for 1 hour or longer. The dilute solution of basal membrane matrix was then removed from each of the wells of the well plate, approximately 1 × 10⁵ fibroblasts suspended in 10% FBS medium were seeded into each well of the well plate, and the fibroblasts were adhesion-cultured.

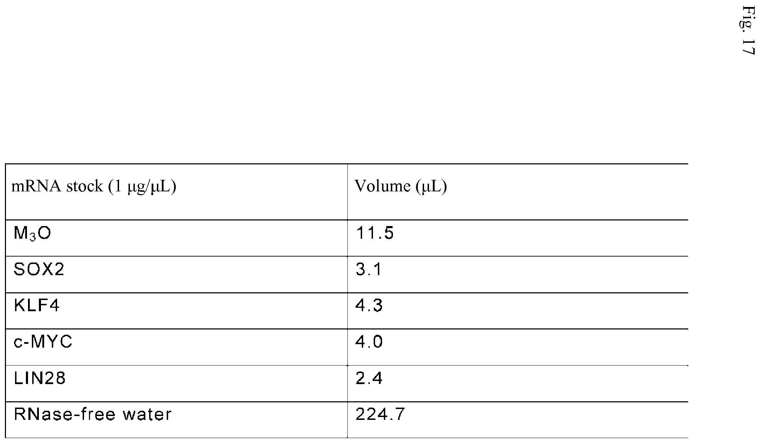

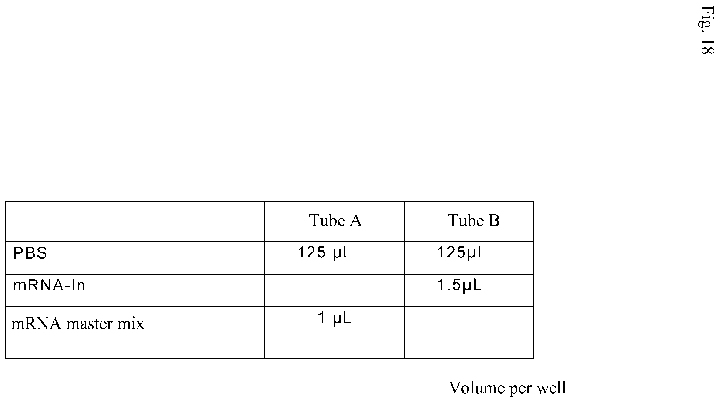

A reprogramming factor mRNA master mix as shown in FIG. 17 was prepared. The medium in each well of the well plate was exchanged with 2 mL of PluriQ® (MTI-GlobalStem). A tube A and a tube B containing the contents listed in FIG. 18 were also prepared. The contents of tube A and tube B were mixed, and the RNA transfection reagent mRNA-In® (Molecular Transfer, Inc.) and the mRNA master mix were combined to form a mixture which was allowed to stand for 10 minutes. The mixture of the RNA-In and mRNA master mix was then added to each well, and the well plate was shaken to disperse the mixture of the RNA-In and mRNA master mix in the medium. The fibroblasts were incubated overnight at 37 °C, 5% CO₂ to transfect the cells with the reprogramming factor RNA.

The cells were continuously transfected with the reprogramming factor RNA for 9 days from the following day, in the same manner. The cells were thus transfected with the reprogramming factor RNA a total of 10 times.

After transfecting the cells with reprogramming factor RNA a total of 10 times, culture medium was removed from each well and the cells were washed with PBS. Next, 300 µL of a cell dissociation solution (TryLE Select® Life Technologies) was added to each well and the well plate was left in an incubator for 10 minutes at 37 °C. Thereafter, 700 µl of PluriQ was added to each well, cell clumps were separated into single cells by pipetting, and a cell suspension was prepared.

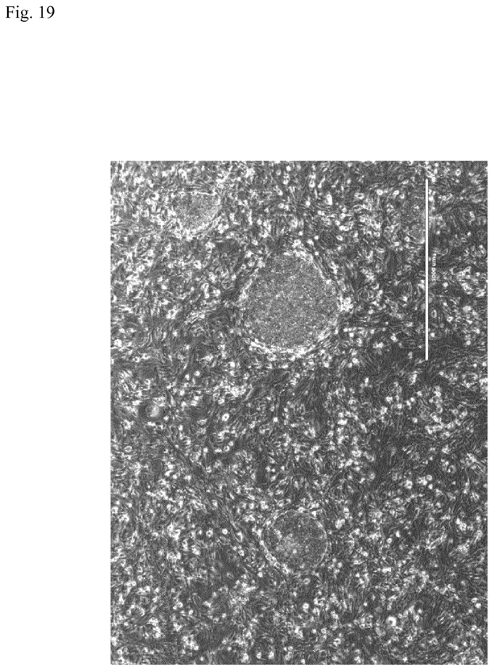

A 0.02% by mass concentration of polymer FP001 was blended into human ES culture medium (Primate ES Cell Medium, ReproCELL) and the gel culture medium was prepared. bFGF was not included in the gel culture medium. FP001 is a culture medium additive consisting mainly of gellan gum. Next, the cell suspension was transferred to a 15 mL tube and was centrifuged at 1000 rpm for five minutes. Furthermore, the supernatant was removed from the 15 mL tube and the cells were resuspended in 2 mL of gel culture medium. Thereafter, 1 mL of gel culture medium was added to a 15 mL tube every other day. Ten days after the cells were transferred to a gel culture medium, the iPS cells prepared in the gel culture medium were reseeded on a feeder, the morphology of the colonies was confirmed, and as can be seen in FIG. 19, the morphology was that of undifferentiated iPS cell colonies.

### (Reference Examples of the second embodiment)

The human blood cells were obtained from a healthy adult male. In addition, modified mRNA (TriLink), a non-adhesive dish, a 15 mL tube, a 50 mL tube, Ficoll, a flow cytometer (BD), anti-CD34 antibodies (Miltenyi Biotec), anti-CD3 antibodies (Miltenyi Biotec), MACS® buffer (Miltenyi Biotec), T cell culture medium, low serum culture medium (Opti-MEM®, Gibco), siRNA transfection reagent (Lipofectamine® RNAiMAX, Thermo Fisher Science), and anti-TRA-1-60 antibodies (BD) were prepared.

The T cell (CD3 positive cells) culture medium was a mixture of the following A culture medium and B culture medium. The A culture medium was a mixture of 15 mL of X vivo-10 (Lonza, 04-743Q) and IL-2 (10 µg/mL). The B culture medium was prepared by mixing 50 µL X vivo-10 and Dynabeads CD3/CD28 (Life Technologies, 111-31D) in a 1.5 mL tube, vortexing for 5 seconds, spinning down, leaving in DynaMag-2 (Thermo fisher Science) for one minute, and removing the supernatant.

Further, a blood cell culture medium (blood stem/progenitor cell culture medium) was prepared by adding 10 µL IL-6 (100 µg/mL), 10 µL SCF (300 µg/mL), 10 µL TPO (300 µg/mL), 10 µL FLT3 ligand (300 µg/mL) and 10 µL IL-3 (10 µg/mL) to 10 mL of serum-free culture medium (StemSpan H3000, STEMCELL Technologies).

Further, solutions containing 100 ng/µL concentrations of OCT3/4 mRNA, SOX2 mRNA, KLF4 mRNA. C-MYC mRNA, LIN28A mRNA, and Green Fluorescent Protein (GFP) were respectively prepared. Next 385 µL of OCT3/4 mRNA-containing solution, 119 µL of SOX2 mRNA-containing solution, 156 µL of KLF mRNA-containing solution, 148 µL of c-MYC mRNA-containing solution, 83 µL of LIN28A mRNA-containing solution and 110 µL of GFP mRNA-containing solution were mixed, and a reprogramming factor mixed solution was obtained. The obtained reprogramming factor mixed solution was dispensed in 50 µl aliquots into 1.5 mL RNase-free tubes (Eppendorf tubes®, Eppendorf) and stored in a freezer at -80 °C.

A centrifuge was set to 18 °C. 5 mL to 50 mL of blood was collected, EDTA was added to the blood and then gently mixed. Also, medium for human lymphocyte separation (Ficoll-Paque PREMIUM, GE Healthcare Japan) was dispensed into each of two 15 mL tubes at 5 mL each. After adding 5 mL of PBS to the blood for dilution, 5 mL thereof was overlaid onto the human lymphocyte separation medium in each of the tubes. During this time, the diluted blood is slowly added onto the medium down the tube wall so as not to disturb the interface.

The solution in the tube was centrifuged for 30 minutes at 18 °C at 400 × g. At this time, acceleration and deceleration were performed slowly. After centrifugation, a white cloudy intermediate appeared in the tube. This white cloudy intermediate layer includes monocytes. The white cloudy intermediate layer in each tube was slowly collected using a Pipetman and transferred to a new 15 mL tube while ensuring the bottom layer was not sucked up. Approximately 1 mL of the white cloudy intermediate layer could be collected from each tube. The intermediate layers of two tubes were combined and transferred to a single tube.

12 mL of PBS was added to the recovered monocytes and the solution was further centrifuged at 200 × g at 18 °C for 10 minutes. The supernatant was thereafter drawn out and removed from the solution using an aspirator, and 3 mL of a serum-free hematopoietic cell medium of known composition (X-VIVO® 10 Lonza) was added to obtain a monocyte suspension. Therefrom, 10 µL of the monocyte suspension was stained with Trypan blue and the count was determined with a hemocytometer.

1 × 10⁷ monocytes were reacted with CD34 antibodies or CD3 antibodies in a 100 µL solution at 4 °C for 15 minutes. After the reaction, 5 mL of MACS® buffer (Miltenyi Biotec) was added and centrifugation was performed at 270 g. After centrifugation, the supernatant was removed, and 1 mL of MACS buffer was added. Thereafter, CD34 positive cells or CD3 positive cells were separated from the monocytes using a separation program of an automatic magnetic cell separator (autoMACS, Miltebyi Biotec).

The 5 × 10⁶ separated monocytes were suspended in 1 mL of T cell culture medium or blood stem/progenitor cell culture medium, seeded into a 12-well plate and cultured. The culture conditions were a CO₂ concentration of 5%, an oxygen concentration of 19%, and a temperature of 37 °C.

100 µL of low serum culture medium (Opti-MEM®, Gibco) and 25 µL of a reprogramming factor mixture were mixed as a first mixture. Further, 112.5 µL of low serum culture medium (Opti-MEM®, Gibco) and 12.5 µL of siRNA transfection reagent (Lipofectamine® RNAiMAX, Thermo Fisher Scientific) were mixed as a second mixture. Thereafter, the first mixture and the second mixture were mixed and left standing at room temperature for 15 minutes to be used as lipofection reaction solution.

60 µL of the obtained lipofection reaction solution was gently added to the 12-well plate in which the monocytes were being cultured, and culturing of the monocytes was continued at 37 °C for 18 hours in a feeder-free medium. The culture conditions were a CO₂ concentration of 5%, an oxygen concentration of 19% and a temperature of 37 °C. The concentration of monocytes when the lipofection reaction solution was added was 3 × 10⁶. 18 hours later, the monocytes were collected in a 15 mL tube, centrifuged at 300 g, and the supernatant was removed. Thereafter 1.25 m of blood cell culture medium for CD 34 was added to a 15 mL tube, the monocyte suspension was returned to the 12-well plate, and the monocytes were cultured feeder free overnight at 37 °C. The culture conditions were a CO₂ concentration of 5% and an oxygen concentration of 19%. The aforementioned step was repeated once every two days for seven days.

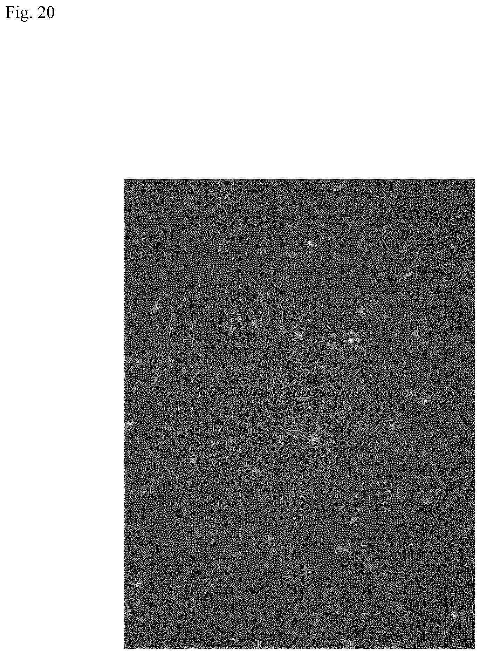

On day 7 from the start of lipofection, the concentration of cells that had been lipofected a total of four times was 3 × 10⁶. A portion of the cells were removed from the 12-well plate to be checked for GFP expression with a fluorescence microscope, and as can be seen in FIG. 20, GFP expression was confirmed. Thus, it was confirmed that mRNA had been transfected into the monocytes and proteins had been synthesized by the transfected mRNAs.

### (Confirmation of TRA-1-60 expression)

On day 7 from the start of lipofection, a portion of the cells were removed from the 12-well plate, and these cells that had been removed were stained with antibodies labelled with allophycocyanin (APC) fluorescent dye, the antibodies being antibodies against TRA-1-60 which is a surface antigen specifically expressed by cells in which reprogramming has started. Thereafter, a fluorescence activated cell sorter (FACS®, BD) was used to confirm the proportion of TRA-1-60 positive cells and thus confirm that cells had started to be reprogrammed, iPS cell genes were being expressed, and that iPS cells would be formed.

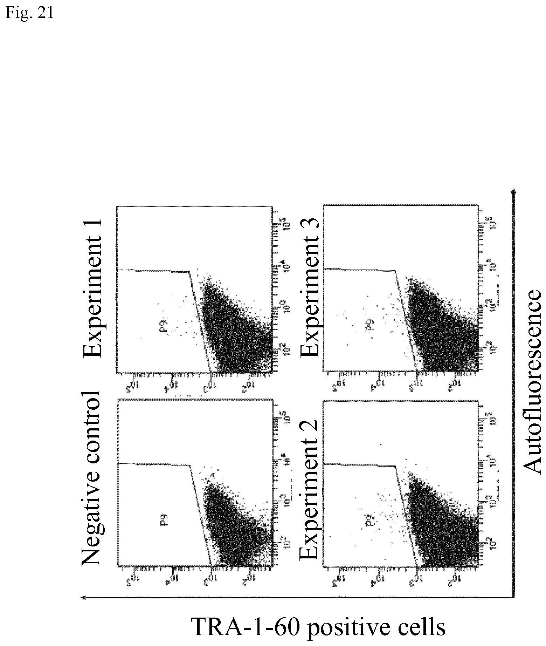

As shown in FIG. 21, a dot plot was created with the x-axis as intensity of autofluorescence and the y-axis as fluorescence intensity of fluorescently labelled anti-TRA-1-60 antibodies. TRA-1-60 positive cells could not be detected in negative controls in which genes were not transfected. In contrast thereto, TRA-1-60 positive cells were detected in Experiments 1, 2 and 3. Note that in Experiment 1 the results were derived from whole monocytes not separated by marker, in Experiment 2 the results were derived from separated CD3 positive cells, and in Experiment 3, the results were derived from separated CD34 positive cells. Thus, it was shown that iPS cells could be induced by transfecting cells derived from blood with reprogramming factors by the lipofection of reprogramming factor RNA.

## Claims

1. A production method for an artificial pluripotent stem cell, comprising:
preparing a somatic cell; and
introducing a Sendai virus comprising a reprogramming factor RNA into the somatic cell.

2. The production method for an artificial pluripotent stem cell according to claim 1, wherein the Sendai virus comprising the reprogramming factor RNA is introduced into the somatic cell which is suspension-cultured in a gel medium.

3. The production method for an artificial pluripotent stem cell according to claim 1, wherein the Sendai virus comprising the reprogramming factor RNA is introduced into the somatic cell which is adhesion-cultured.

4. The production method for an artificial pluripotent stem cell according to any one of claims 1 to 3, further comprising reprogramming the somatic cell in a gel culture medium.

5. The production method for an artificial pluripotent stem cell according to any one of claims 1 to 4, wherein the somatic cell is a fibroblast.

6. The production method for an artificial pluripotent stem cell according to any one of claims 1 to 4, wherein the somatic cell is a blood cell.

7. The production method for an artificial pluripotent stem cell according to claim 6, further comprising separating a monocyte as the somatic cell using an erythrocyte separating agent.

8. The production method for an artificial pluripotent stem cell according to claim 6, further comprising separating a monocyte as the somatic cell using a filter.

9. The production method for an artificial pluripotent stem cell according to claim 6, further comprising separating a monocyte as the somatic cell using an immunomagnetic bead.

10. The production method for an artificial pluripotent stem cell according to any one of claims 1 to 9, wherein the reprogramming factor RNA comprises M₃O or OCT3/4 mRNA, SOX2 mRNA, KLF4 mRNA, and c-MYC mRNA.

11. The production method for an artificial pluripotent stem cell according to claim 2 or 4, wherein the gel culture medium is not stirred.

12. The production method for an artificial pluripotent stem cell according to claim 2 or 4, wherein the gel culture medium does not contain a growth factor.

13. The production method for an artificial pluripotent stem cell according to claim 2 or 4, wherein the gel culture medium contains a growth factor at a concentration of 40% by mass or less.

14. The production method for an artificial pluripotent stem cell according to claim 2 or 4, wherein the gel culture medium does not comprise bFGF.

15. A production method for an artificial pluripotent stem cell, comprising:
preparing a somatic cell;
introducing reprogramming factor RNA into the somatic cell using an RNA transfection reagent; and
reprogramming the somatic cell in a gel culture medium.

16. The production method for an artificial pluripotent stem cell according to claim 15, wherein the somatic cell is a fibroblast.

17. The production method for an artificial pluripotent stem cell according to claim 15, wherein the somatic cell is a blood cell.

18. The production method for an artificial pluripotent stem cell according to claim 17, further comprising separating a monocyte as the somatic cell using an erythrocyte separating agent.

19. The production method for an artificial pluripotent stem cell according to claim 17, further comprising separating a monocyte as the somatic cell using a filter.

20. The production method for an artificial pluripotent stem cell according to claim 17, further comprising separating a monocyte as the somatic cell using an immunomagnetic bead.

21. The production method for an artificial pluripotent stem cell according to any one of claims 15 to 20, wherein the reprogramming factor RNA comprises M₃O or OCT3/4 mRNA, SOX2 mRNA, KLF4 mRNA, and c-MYC mRNA.

22. The production method for an artificial pluripotent stem cell according to any one of claims 15 to 21, wherein the gel culture medium is not stirred.

23. The production method for an artificial pluripotent stem cell according to any one of claims 15 to 22, wherein the gel culture medium does not comprise a growth factor.

24. The production method for an artificial pluripotent stem cell according to any one of claims 15 to 23, wherein the gel culture medium contains a growth factor at a concentration of 40% by mass or less.

25. The production method for an artificial pluripotent stem cell according to any one of claims 15 to 24, wherein the gel culture medium does not comprise bFGF.

26. The production method for an artificial pluripotent stem cell according to any one of claims 15 to 25, wherein the introduction of the reprogramming factor RNA is carried out a plurality of times.
